# FASCICULE DE BREVET EUROPEEN

(11) **EP 1 237 836 B1**
(45) Date de publication et mention de la délivrance du brevet: **06.05.2004**
(21) Numéro de dépôt: 00987558.4
(22) Date de dépôt: 14.12.2000
(51) Int. Cl.: C07C 45/53, C07C 29/132, C07C 409/14, C07C 49/403, C07C 35/08, B01J 31/16, B01J 31/18

(54) **PROCEDE DE PREPARATION DE MELANGES ALCOOLS/CETONES**
VERFAHREN ZUR HERSTELLUNG VON MISCHUNGEN VON ALKOHOLEN/KETONEN
METHOD FOR PRODUCING ALCOHOL/KETONE MIXTURES

(30) Priorité: 17.12.1999 FR 9916010
(43) Date de publication de la demande: 11.09.2002
(73) Titulaire: Rhodia Polyamide Intermediates, 69192 Saint-Fons (FR)
(72) Inventeur: CLARK, James, York, Yorkshire Y010 3HW (GB); FACHE, Eric, F-69300 Caluire et Cuire (FR); MACQUARRIE, Ducan, York, Yorkshire (GB); PRICE, Peter, Ilkley, Yorkshire (GB); RAFELT, John, Blandford Forum, Dorset DT11 0TL (GB)
(74) Mandataire: Esson, Jean-Pierre
(86) Numéro de dépôt international: PCT/FR2000/003517
(87) Numéro de publication internationale: WO 2001/044153

(56) Documents cités:
- EP-A- 0 367 326
- EP-A- 0 428 803
- EP-A- 0 453 021
- WO-A-92/16487
- CHISEM, IAN C. ET AL: "Catalytic oxidation of alkyl aromatics using a novel silica supported Schiff base complex" CHEM. COMMUN. (CAMBRIDGE) (1998), (18), 1949-1950, XP002149029 cité dans la demande

## Description

L'invention concerne un procédé de préparation d'un mélange alcools/cétones par décomposition d'un hydropéroxyde d'alkyle.

Elle se rapporte plus particulièrement à la préparation d'un mélange cyclohexanol/cyctohexanone par décomposition d'hydropéroxyde de cyclohexyle en présence d'une catalyse hétérogène.

Parmi ces hydroperoxydes organiques, l'hydroperoxyde de cyclohexyle est préparé par oxydation du cyclohexane. Par décomposition catalytique, il conduit à la cyclohexanone et au cyclohexanol.

Les procédés industriels de production de ces mélanges cyclohexanol/cyclohexanone sont d'une importance économique très grande car ils permettent l'accès à la fabrication de produits chimiques de grandes importances, tels que l'acide adipique. Ces procédés sont décrits dans une abondante littérature tant articles scientifiques que brevets.

Le procédé industriel classique consiste en une oxydation du cyclohexane par l'air permettant d'obtenir un mélange de composés dont l'hydropéroxyde de cyclohexyle (HPOCH), des alcools, cétones et acides. L' hydropéroxyde de cyclohexyle est transformé en un mélange cyclohexanol/cyclohexanone par différentes réactions telles qu'hydrogénation ou décomposition.

La décomposition des hydroperoxydes organiques et notamment de l'hydroperoxyde de cyclohexyle (HPOCH) peut tout d'abord être réalisée par catalyse homogène, c'est-à-dire en présence d'un catalyseur dissous dans le milieu. réactionnel. Ainsi le brevet FR-A-1 580 206 décrit l'oxydation d'un cycloalcane en phase liquide suivie du chauffage de la solution de l'hydroperoxyde de cycloalkyle dans le cycloalcane ainsi obtenue, en présence d'un dérivé soluble du chrome comme catalyseur. De même les articles de Journal of the American Chemical Society (1985), 107, pages 3534 à 3540 ou de Journal of Molecular Catalysis (1988), 48, pages 129 à 148, décrivent l'utilisation de sels organiques, tels que l'octanoate de cobalt ou de complexes dissous dans la phase liquide organique où se déroule la réaction ou dans une phase aqueuse en contact avec ladite phase organique.

Cette décomposition de l'hydropéroxyde de cyclohexyle peut être également réalisée par neutralisation des acides présents dans le milieu avec un hydroxyde alcalin et en présence de sels métalliques tels que décrits dans les brevets US 4,720,592 et 4,238,415. Toutefois, le rendement de production du mélange cyclohexanollcyclohexanone n'est pas très élevé et de nombreux sous-produits sont également formés.

Le brevet US 3,925,316 décrit un procédé de décomposition d'hydropéroxyde de cyclohexyle en présence de catalyseurs homogènes constitués par des composés solubles de vanadium, ruthénium ou molybdène. D'autres systèmes catalytiques à base de couple de différents métaux présents sous forme de composés solubles sont décrits par exemple dans les brevets US 3,401,193 ; 3,987,100 ; 4,551,553.

La décomposition des hydroperoxydes en présence d'un catalyseur homogène présente un certain nombre d'inconvénients. Ainsi d'importantes quantités de catalyseur sont entraînées et se retrouvent finalement soit dans le produit préparé, soit dans les effluents. Il n'est pas facile de récupérer ce catalyseur et il est donc nécessaire de rajouter du catalyseur neuf. En outre, la présence de métaux, essentiellement des métaux lourds, dans les effluents n'est pas très favorable à l'environnement et il est indispensable de l'éviter au maximum.

Il a été proposé pour tenter de pallier ces inconvénients, de réaliser cette décomposition par catalyse hétérogène, c'est-à-dire en présence d'un catalyseur non dissous dans le milieu réactionnel.

Ainsi, le brevet US 4,173,587 décrit l'utilisation d'un composé non soluble de rhénium pour la décomposition de l'hydropéroxyde de cumène.

Le brevet EP-A-0 492 807 décrit également, la préparation de phénol et d'acétone à partir d'hydroperoxyde de cumyle, en présence d'un catalyseur zéolithe de type mordénite ou faujasite choisi parmi les zéolithes Y, les zéolithes Y désaluminées, stabilisées thermiquement, les zéolithes Y échangées par des terres rares, notamment par des sels de lanthane ou par des métaux de transition, notamment par des sels de cobalt ou de nickel, et les zéolithes Y traitées par des fluorures.

Dans ces cas, les métaux ne sont également pas suffisamment fixés sur le support et il se produit une dissolution pa rtielle dans le milieu réactionnel lors de la mise en oeuvre des catalyseurs.

Le brevet US 4,543,427 décrit la préparation d'un mélange cyclohexanol/cyclohexanone consistant à traiter un hydropéroxyde de cyclohexyle par un catalyseur supporté contenant de 2 à 30 % en poids exprimé en élément cobalt, d'un oxyde de cobalt déposé ou absorbé sur un support zéolithe. Ce catalyseur n'est pas stable et une quantité importante du composé métallique se dissout dans le milieu réactionnel. On se trouve alors confronté aux problèmes évoqués précédemment pour la catalyse homogène

Avant cela, le brevet US 2,851,496 a décrit l'utilisation des métaux du groupe VIII comme le cobalt déposé sur une alumine, une silice, du charbon ou du kieselguhr comme catalyseurs de décomposition de l'hydropéroxyde de cyclohexyle. Toutefois, ce catalyseur a une durée de vie réduite.

Le brevet Ep 659726 décrit un procédé de préparation d'un mélange alcool/cétone par décomposition d'un hydropéroxyde d'alkyle en présence d'un métal immobilisé sur un support en présence d'une phase aqueuse et d'un composé basique. Le support est un oxyde métallique tel que TiO₂ ou ZrO₂ sur lequel est déposé un composé de manganèse, fer, cobalt, nickel ou cuivre.

Le brevet US 5,298,665 décrit également l'utilisation d'un catalyseur constitué par un composé métallique déposé ou fixé sur un support. Comme composé métallique les composés des métaux suivants : cobalt, chrome, vanadium, molybdène, ruthénium, titane, manganèse et fer sont cités. Le support est un oxyde métallique choisi parmi la silice, l'alumine, l'oxyde de titane. Ce support comprend à sa surface des groupements aminés aromatiques ou aliphatiques. Ce catalyseur est utilisé pour transformer un hydropéroxyde d'alkyle en un mélange d'alcool et de cétone.

Les catalyseurs décrits ci-dessus présentent une durée de vie limitée car dans la plupart des cas, l'élément métallique est partiellement dissous dans le milieu, la catalyse étant principalement effectuée par la fraction dissoute. Le catalyseur supporté s'épuise en métal catalytiquement actif et le mélange de cétones/alcools produit comprend comme impureté gênante la fraction dissoute de métal.

La demande de brevet WO-A-94/08932 propose, pour remédier aux inconvénients des catalyseurs hétérogènes mentionnés précédemment, d'effectuer la décomposition des hydroperoxydes organiques en présence d'un tamis moléculaire contenant des oxydes d'aluminium et/ou de silicium et/ou de phosphore et un métal catalyseur incorporé dans la matrice cristalline dudit tamis moléculaire. Il semble que le métal actif de ces catalyseurs hétérogènes n'élue pratiquement pas. Cependant si le problème de l'élution du catalyseur dans le milieu réactionnel semble ainsi résolu, il ressort de la demande de brevet elle-même que le catalyseur se désactive rapidement, ce qui induit la nécessité d'une réactivation par séparation du catalyseur et calcination. Dans le cadre d'une exploitation industrielle d'un tel procédé, il est clair qu'il est prohibitif de devoir fréquemment séparer le catalyseur du milieu réactionnel pour le réactiver.

Un des buts de la présente invention est de remédier à ces inconvénients en proposant un procédé de fabrication d'un mélange d'alcools et de cétones à partir d'un hydropéroxyde d'alkyle comprenant une catalyse hétérogène dans lequel la durée de cycle et durée de vie du catalyseur sont élevées et avec production de mélange alcool/cétone ne comprenant pas ou une très faible quantité d'élément métallique utilisé comme catalyseur.

A cet effet, l'invention propose un procédé de fabrication d'un mélange d'alcools et/ou de cétones par décomposition d'un hydropéroxyde d'alkyle en présence d'un catalyseur comprenant un élément métallique catalytiquement actif immobilisé sur un support solide, ledit élément métallique étant choisi dans le groupe comprenant les éléments appartenant aux groupes IB à VIIB ou VIII de la classification périodique de MENDELEEV (version CAS) incluant la famille des lanthanides caractérisé en ce que ledit élément métallique est présent à la surface dudit support solide sous forme, d'un fragment organométallique de formules (I) ou (II). 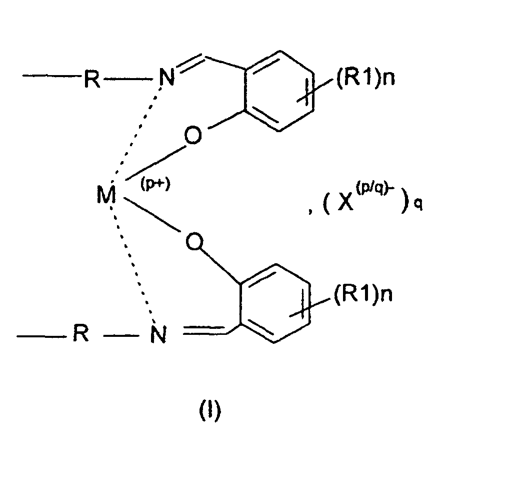 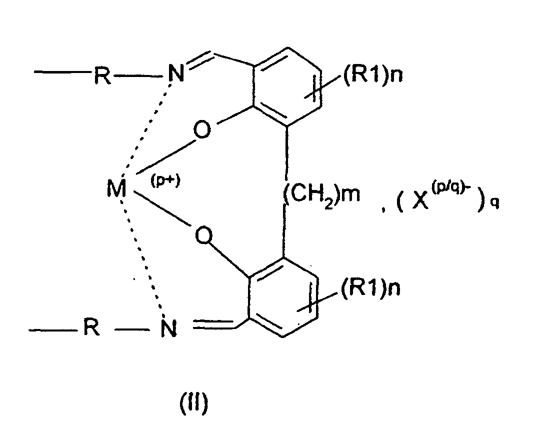 dans lesquelles :
M est un ion métallique ou une combinaison d'ions métalliques correspondants aux éléments appartenant aux groupes IB à VIIB ou VIII de la classification périodique de MENDELEEV (version CAS), incluant les lanthanides,
R est un radical hydrocarboné linéaire ou ramifié comprenant de 1 à 12 atomes de carbone.
R₁ est un atome d'halogène un radical hydroxyl, alcoxy, carboxyl, amine ou hydrocarboné aliphatique, arylaliphatique, aromatique, alkylaliphatique pouvant comprendre des hétéroatomes,
X est un anion
n est un nombre entier compris entre 0 et 4
m est un nombre entier compris entre 1 et 6
p et q sont des nombre entiers compris entre 0 et 4

Dans un mode de réalisation préféré de l'invention, l'ion métallique M correspondant aux éléments choisis dans le groupe préférentiel suivant :chrome, cobalt, cuivre, fer, manganèse, titane, vanadium, molybdène, ruthénium l'or, l'osmium Les éléments préférés de l'invention sont le chrome et le cuivre.

Les anions convenables pour l'invention sont généralement les anions formant un sel soluble avec le métal M dans le milieu de préparation du complexe. A titre d'exemple, on peut citer les anions carboxyliques, tels que les oxalates, acétates, les halogénures, sulfonates ou leurs mélanges.

De même, le support est, de préférence, un composé minéral tel que les oxydes minéraux comme l'alumine, la silice, l'oxyde de titane, l'oxyde de zirconium, les oxydes de terres rares comme l'oxyde de cérium ou l'oxyde de lanthane ou des composés minéraux comme le phosphate de lanthane. Le support peut également être choisi parmi les zéolithes, tamis moléculaires de type MCM, HMS par exemple ou les supports synthétisés à partir de polymères fonctionnalisés tels que polystyrènes, polyacrylonitriles, par exemple. De manière plus générale, toutes structures poreuses solides peuvent convenir pour la présente invention.

Comme catalyseurs conformes à l'invention, on peut citer notamment ceux décrits dans l'article de J.H. Clark et al "Catalytic oxidation of alkyl aromatic using a novel silica supported Schiff base complex" publié dans la revue J. Chem.Commun 1998 pages 1941-1950.

Ces catalyseurs sont obtenus préférentiellement par un procédé en solution dans lequel le complexe métallique préformé au préalable est fixé sur la surface du support et plus particulièrement de la silice, dans la dernière étape de synthèse. Ce procédé de synthèse présente notamment l'avantage de former de manière plus simple un complexe métallique en utilisant un solvant convenable.

Le support est ensuite ajouté dans le milieu où a été formé le complexe pour permettre à ce dernier de se fixer sur ledit support.

Comme exemple de complexe métallique, on peut citer ceux répondant aux formules générales (III) et (III)ₐ :ci-dessous :

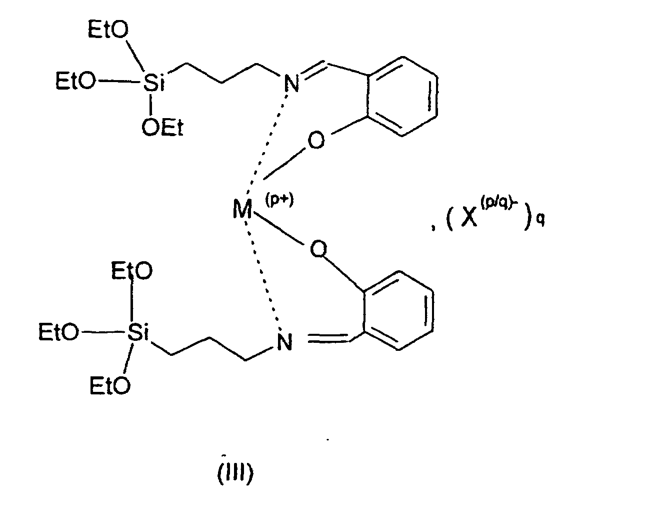

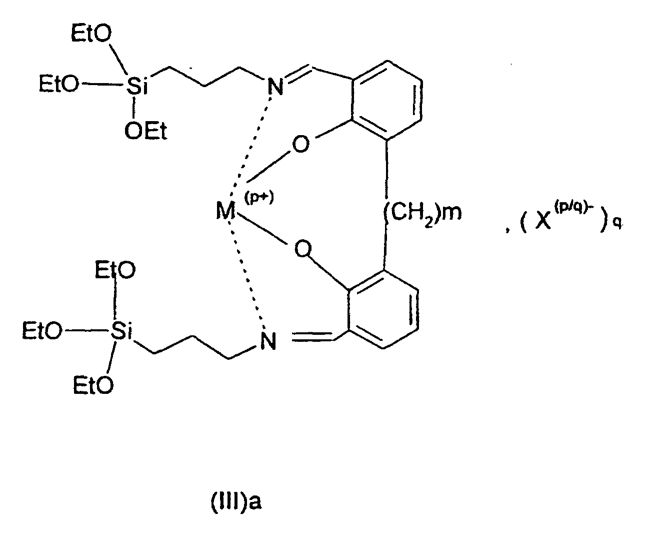

Dans ces formules m, p,q ont les significations indiquées précédemment.

Le catalyseur préparé selon ce procédé présente une meilleure activité catalytique que ceux formés par fixation du ligand sur le support puis dans une dernière étape, addition des ions métalliques pour former le complexe.

Un autre mode de réalisation d'un catalyseur conforme à l'invention consiste à ajouter dans un milieu liquide un précurseur d'un gel du support solide et les composés nécessaires pour la formation du complexe métallique décrit ci-dessus. Le milieu est ensuite modifié pour provoquer la formation du gel, par exemple par modification du pH ou ajout d'un cosolvant. Cette modification du milieu peut être obtenue lors de l'addition de l'un des composés nécessaires à la formation du complexe.

Ces procédés de synthèse sont décrits dans l'article indiqué ci-dessus et ne sont donnés qu'à titre d'exemple et d'illustration.

Comme exemple de catalyseur ainsi synthétisé, on peut citer le catalyseur dont le fragment organométallique a la formule (IV) suivante :

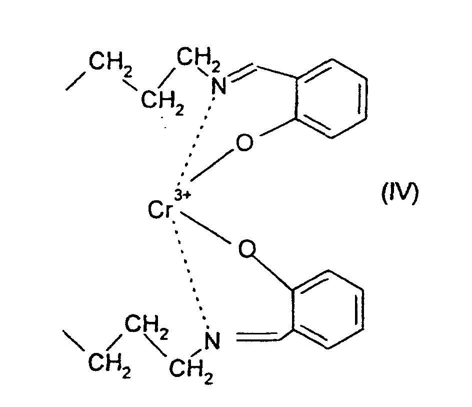

Ces catalyseurs utilisés dans le procédé de décomposition d'hydropéroxydes d'alkyles présentent de très bonnes activité et sélectivité en alcools et cétones. Ces activité et sélectivité sont supérieures à celles observées avec un catalyseur homogène au chrome.

En outre, la durée de vie du catalyseur est longue car il n'a pas été mis en évidence de phénomène de désactivation.

Enfin, la quantité de métal élué au cours de la réaction de décomposition est très faible ce qui permet d'une part de réduire la quantité de métal à engager et d'autre part de limiter la présence de polluant métallique dans le mélange cétone/alcool produit ou dans les rejets du procédé.

L'invention s'applique notamment à la décomposition des hydropéroxydes d'alkyles produits par oxydation à l'air d'un alcane comprenant de 3 à 30 atomes de carbone.

Cette réaction est généralement réalisée en milieu solvant.

L'oxydation des alcanes est réalisée dans des conditions classiques et largement décrites dans la littérature. A titre d'illustration, cette oxydation peut être effectuée en phase liquide en présence d'oxygène pure, d'air ou d'un mélange enrichi en oxygène à une température comprise entre 80°C et 250°C.

En général, dans les procédés d'oxydation des alcanes, le taux de transformation de ceux-ci est compris entre 1 et 50 % en poids environ.

Cette oxydation peut être réalisée en absence de catalyseur ou avec un catalyseur d'oxydation choisi parmi les métaux de transition tels que, le cobalt, le chrome, le manganèse, le fer, le nickel, le cuivre ou un mélange de ceux-ci.

Généralement, l'hydropéroxyde d'alkyle formé est soluble dans l'alcane correspondant qui est utilisée comme solvant. Toutefois, il est possible d'utiliser d'autres solvants tels que les alcools, les cétones et en particulier les alcools et cétones produites par la réaction de décomposition de l'hydroperoxyde.

La réaction de décomposition de l'hydropéroxyde d'alkyle est ensuite réalisée en présence du catalyseur supporté décrit précédemment. Ce catalyseur est mis en oeuvre sous forme de lit fixe ou fluidisé ou en suspension dans le milieu. Cette décomposition peut être mise en oeuvre directement dans le milieu résultant de l'oxydation, après lavage à l'eau dudit milieu réactionnel ou après extraction de l'hydropéroxyde.

La température de la réaction de décomposition est maintenue entre 25 et 200°C, de préférence entre 70 et 150°C environ, par exemple à la température d'évaporation de l'alcane ou du solvant, avec reflux de celui-ci.

La quantité de catalyseur engagé dépend du mode de réalisation. Ainsi, en lit fixe la quantité de catalyseur est plus élevée que celle utilisée dans l'utilisation d'un catalyseur en suspension.

De même, la concentration en hydropéroxyde peut varier dans de larges limites.

Les produits obtenus sont principalement des alcools et des cétones qui peuvent être séparés et extraits du milieu réactionnel par distillation, si nécessaire.

Il est également possible de soumettre le milieu réactionnel à une nouvelle oxydation par exemple avec l'acide nitrique pour fabriquer l'acide adipique.

Ce milieu réactionel peut également être employé comme matière première pour la synthèse du caprolactame.

Les alcanes qui peuvent être oxydés sont notamment les alcanes linéaires ou ramifiés, les cycloalcanes comprenant de 3 à 30 atomes de carbone.

On peut citer, à titre d'exemple, le propane, le cyclohexane, le cycloheptane, le méthylbenzène, éthylbenzène, phénylcyclohexane, diphénylméthane, phénylcyclododecane, cydododécane, 1,2-dicyclohexylméthane, le cumène, l'isobutane, 2-méthytpropane, 2-propyl benzène, cyclohexène, 4-tert.butyl-1-cycloheptylbenzène, 2-isopropylnaphtalène, fluorène, ou 1,8-diméthylfluorène.

Le procédé s'applique plus particulièrement à l'oxydation du cyclohexane et la décomposition de l'hydropéroxyde de cyclohexyle en cyclohexanone/cyclohexanol.

Il s'applique également à la décomposition d'un hydropéroxyde d'alkyle obtenu par d'autres procédés que celui d'oxydation d'un alcane.

L'invention sera mieux illustrée au vu des exemples donnés ci-dessous uniquement à titre indicatif.

Trois catalyseurs A, B, C fabriqués selon les procédés décrits dans les exemples 1 à 3.

Ces catalyseurs comprennent un complexe du chrome forment un fragment organométallique fixé sur un support silice.

### Exemple 1 : Préparation du catalyseur A

De l'aldéhyde salicylique est ajouté dans de l'éthanol avec du 3-aminopropyl(triméthoxy)silane. La solution devient instantanément jaune. De l'acétate de chrome est ajouté à la solution et le mélange est agité pendant 30 minutes pour permettre la formation du complexe de formule suivante :

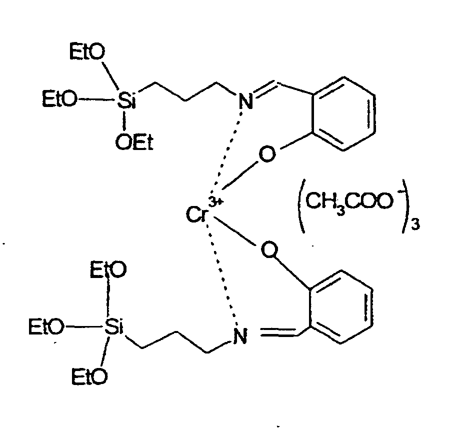

De la silice commercialisée sous le nom KIESELGEL 100 est ajouté dans le milieu qui est maintenu sous agitation pendant une nuit.

Le produit final est extrait par fixation et est lavé par de l'eau et de l'alcool.

Le produit est séché à 70°C pendant deux heures.

Le catalyseur obtenu présente un diamètre moyen de pore de 100 □ et des particules de taille comprise entre 30 et 140µm.

La concentration molaire de chrome est de 0,106mmol par gramme de catalyseur (0,57% en poids)

### Exemple 2 : Préparation du catalyseur B

L'exemple 1 est répété mais en utilisant une silice mésoporeuse HMS comme support à la place de la silice KIESELGEL.

La silice mésoporeuse HMS est obtenue selon le procédé suivant :

0,054 mol de dodécylamine sont ajoutés dans un mélange eau/éthanol (11,8 mol d'eau et 3,6 mol d'éthanol). La solution est agitée pendant 20 minutes, avec addition de 0,4 mol de tétraéthoxysilane. Le précipité blanc obtenu après mûrissement de 18 heures est filtré, séché et calciné à une température de 600°C pendant 4 heures pour éliminer les composés organiques. D'autres amines peuvent être utilisées comme la décylamine

Le catalyseur obtenu contient 0,36mmol de chrome par gramme de catalyseur (1,80% en poids)

### Exemple 3 : Préparation du catalyseur C par une méthode sol-gel

0,02 mol d'aldéhyde salicylique et 0,02 mol d'aminopropyl(triméthoxy)silane sont ajoutés dans de l'éthanol pur. 10 mmol d'acétate de chrome sont ajoutés dans la solution maintenue sous agitation pendant 30 minutes. Une autre solution est préparée par addition de 0,049 mol de dodécylamine dans 100 ml d'eau. De l'éthanol pur est ajouté progressivement dans cette dernière solution jusqu'à dissolution complète du dodécylamine.

Les deux solutions ainsi préparées sont mélangées avec addition immédiate de tétraéthoxysilane (0,255mol). Le mélange obtenu est maintenu sous agitation pendant une nuit.
Le solide vert formé est filtré et lavé avec de l'eau et de l'éthanol. Le solide ainsi, obtenu est traité avec de l'éthanol puis séché à 70°C pour éliminer le solvant.
Le catalyseur obtenu comprend 0,073mmol de Cr par gramme de catalyseur (0,52% en poids)

### Exemple 4 : Décomposition d'hydropéroxyde de cyclohexyle (HPOCH)

La solution à traiter est issue de l'oxydation par l'air à 180°C du cyclohexane. Elle contient 6,09% d'hydroperoxyde de cyclohexyle, 1,36 % en poids de cycolhexanone et 1,37% en poids de cyclohexanol.

40g de cette solution sont chauffés à 80°C avec distillation azéotropique de l'eau, en présence de 1g de catalyseur.

La nature du catalyseur ajouté et la durée de réaction sont indiquées dans le tableau 1 ci-dessous ainsi que les rendements de transformation et de production des différents composés chimiques.

Les différentes concentrations en cyclohexanone, cyclohexanol et HPOCH sont déterminées par les méthodes suivantes :
- Dosage de l'hydroperoxyde de cyclohexyle :
   Le principe est l'oxydation de l'iodure de potassium par l' hydroperoxyde et dosage en retour de l'iode formée par une solution de thiosulfate de sodium.
- Dosage des concentrations en cétone et alcool:
   Le dosage est réalisé par une méthode chromatographique après réduction de l'hydroperoxyde présent en alcool par réaction avec une triphénylphosphine.
   L'analyse chromatographique donne la concentration totale en cétones et celle en alcools.
   La concentration en cétone formée et celle en alcool formée sont calculées en prenant en compte les concentrations initiales en cétone, alcool et celle de l'hydroperoxyde résiduel.

Dans le tableau I ci-dessous :
- TT signifie le taux de transformation de l'hydropéroxyde de cyclohexyle en %;
- RTone signifie la sélectivité de la réaction en composés cétones (exprimé en quantité de cétone exprimée en cyclohexanone formée par la décomposition de l'HPOCH par rapport à la quantité théorique de cyclohexanone formée calculée à partir de la quantité réelle de HPOCH transformée);
- RTol signifie le rendement en composés alcool (exprimé en quantité d'alcool exprimée en cyclohexanol formé par la décomposition de l'HPOCH par rapport à la quantité théorique de cyclohexanol formé calculée à partir de la quantité réelle de HPOCH transformée);
- One/Ol : rapport molaire de cétone/alcool.

| **Essai** | **Catalyseur** | **Durée** **(min)** | **TT** | **Rtone** | **Rtol** | **One/Ol** |
|---|---|---|---|---|---|---|
| 4 | A | 70 | 98,8 | 65,0 | 51,5 | 1,26 |
| 5 | B | 60 | 98,5 | 66,4 | 44,8 | 1,5 |
| 6 | C | 60 | 99,1 | 67,2 | 37,4 | 1,8 |

A titre comparatif, un essai a été réalisé avec un catalyseur homogène à base de chrome (le chromate de ditertiobutyle) avec un rapport HPOCH/Cr de 6550. Un taux de transformation (TT) de 94% a été atteint après 90 minutes avec une sélectivité en cyclohexanone élevée (RTone = 81,6% ; RToI = 26,6% ; rapport one/ol = 3,1).

Par ailleurs, une analyse du chrome éludé dans le milieu réactionnel montre que les concentrations en Cr sont très faibles(inférieures au ppm) dans les exemples 4 à 6 (catalyseurs A,.B, C de l'invention).

### Exemple 7 :

Le catalyseur A utilisé dans l'exemple 4 a été récupéré après les 70 minutes de réaction. Un nouvel essai de décomposition d'hydroperoxyde de cyclohexyle a été réalisé selon les conditions de l'exemple 4 mais en utilisant comme catalyseur le catalyseur récupéré. Les informations sur l'avancement de la réaction sont données dans le tableau II ci-dessous :

| **Essai** | **Catalyseur** | **Durée** **(min)** | **TT** | **Rtone** | **Rtol** |
|---|---|---|---|---|---|
| 7 | A récupéré | 5 | 47,6 | | |
| | | 40 | 97,7 | | |
| | | 60 | 98,5 | 66,4 | 44,8 |
| 4 | A | 5 | 9,4 | | |
| | | 30 | 53,8 | | |
| | | 70 | 98,8 | 65 | 51,5 |

En outre dans l'exemple 4 ci-dessus la concentration en chrome élué est de l'ordre de 0,7mg/kg de solution soit 0,47% du chrome engagé est élué. Dans l'exemple 7 cette concentration est inférieure à 0,1 mg/ Kg de solution soit un pourcentage de chrome élué inférieur à 0,07%.

## Revendications

1. Procédé de fabrication d'un mélange cétanes/alcools par décomposition d'un hydropéroxyde d'alkyle en présence d'un catalyseur comprenant un élément métallique catalytiquement actif immobilisé sur un support solide, **caractérisé en ce que** le catalyseur comprend un fragment organométallique de formules générales I ou II 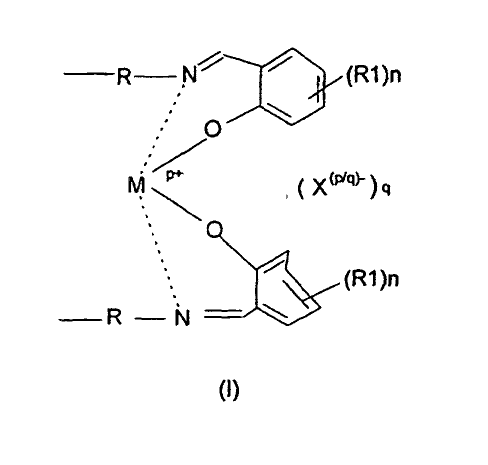 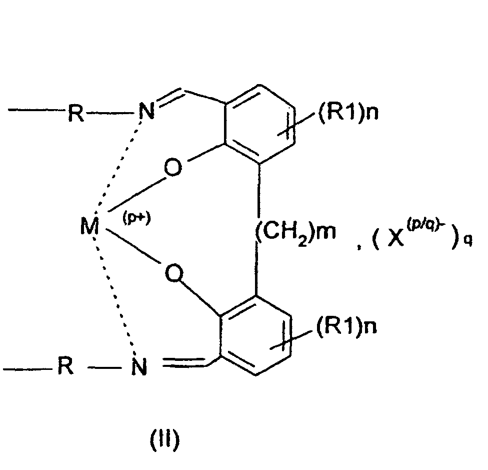 dans lesquelles :
M est un ion métallique ou une combinaison d'ions métalliques correspondants aux éléments appartenant aux groupes IB à VIIB ou VIII de la classification périodique de MENDELEEV (version CAS) incluant les lanthanides
R est un radical hydrocarboné linéaire ou ramifié comprenant de 1 à 12 atomes de carbone.
R₁ est un atome d'halogène un radical hydroxyle, alcoxy, carboxyle, amine ou un radical hydrocarboné aliphatique, arylaliphatique, aromatique, alkylaromatique pouvant comprendre des hétéroatomes,
X représente un anion
n est un nombre entier compris entre 0 et 4
m est un nombre entier compris entre 1 et 6.
p et q sont des nombres entiers compris entre 0 et 4

2. Procédé selon la revendication 1, **caractérisé en ce que** l'élément métallique M est choisi dans le groupe comprenant le chrome, le cobalt, le fer, le manganèse, le titane, le cuivre, le vanadium, le molybdène, le ruthénium, l'or, l'osmium.

3. Procédé selon la revendication 1, **caractérisé en ce que** le support solide est un oxyde minéral choisi dans le groupe comprenant l'alumine, la silice, l'oxyde de zirconium, les oxydes de terres rares, l'oxyde de titane, ou des composés minéraux comme le phosphate de lanthane ou des zéolithes, des tamis moléculaires ou des supports synthétisés à partir de polymères fonctionnalisés.

4. Procédé selon l'une des revendications précédentes, **caractérisé en ce que** le catalyseur est obtenu par fabrication d'un complexe métallique de formules générales III ou IIIa: 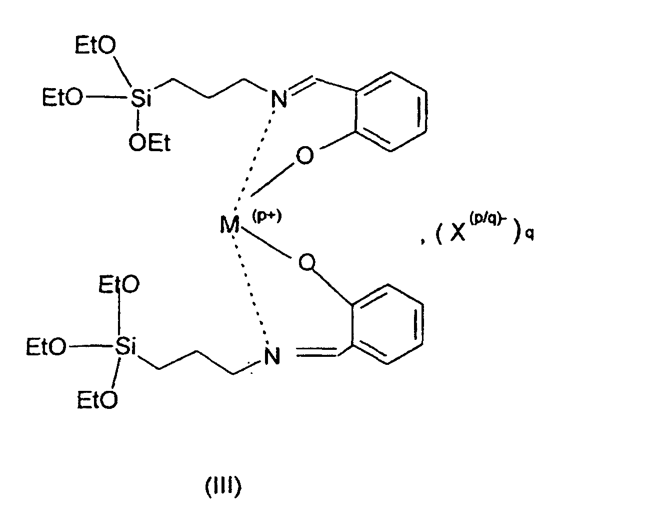 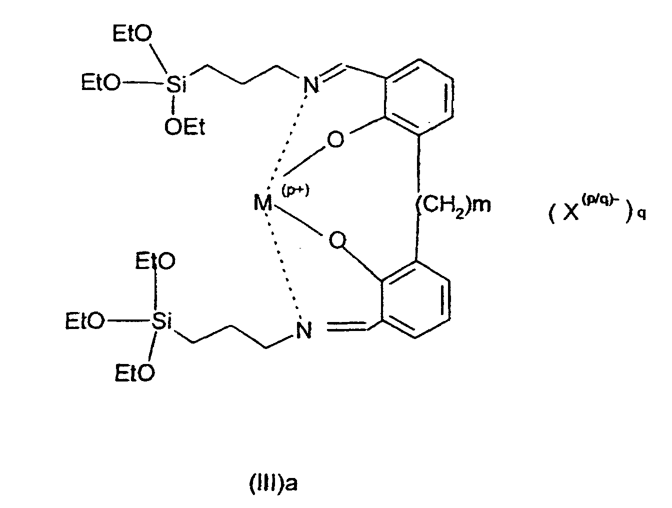 dans un solvant et greffage du complexe sur le support par mise en contact du complexe et du support.

5. Procédé selon la revendication 4, **caractérisé en ce que** le support solide est produit in situ dans la solution sous forme de gel

6. Procédé selon l'une des revendications précédentes **caractérisé en ce que** l'hydropéroxyde d'alkyle est obtenu par oxydation d'un alcane par un agent oxydant.

7. Procédé selon la revendication 6 **caractérisé en ce que** l'agent oxydant est choisi parmi l'oxygène, l'air ou les mélanges enrichis en oxygène.

8. Procédé selon la revendication 6 ou 7, **caractérisé en ce que** l'alcane comprend de 3 à 30 atomes de carbone et est choisi dans le groupe comprenant le propane, le cyclohexane, le cycloheptane, le méthylbenzène, éthylbenzène, phénylcyclohexane, diphénylméthane, phénylcyclododecane, cyclododécane, 1,2-dicyclohexylméthane, le cumène, l'isobutane, 2-méthylpropane, 2-propyl benzène, cyclohexène, 4-tert.butyl-1-cycloheptylbenzène, 2-isopropylnaphtalène, fluorène, ou 1,8-diméthylfluorène.

9. Procédé selon l'une des revendications précédentes **caractérisé en ce qu'**il comprend une étape de production d'hydroperoxyde de cyclohexyle par oxydation du cyclohexane, et décomposition de l'hydropéroxyde en cyclohexanone et/ou cyclohexanol.

## Claims

1. Process for the manufacture of a ketone/alcohol mixture by decomposition of an alkyl hydroperoxide in the presence of a catalyst comprising a catalytically active metal element immobilized on a solid support, **characterized in that** the catalyst comprises an organometallic part of general formulae I or II 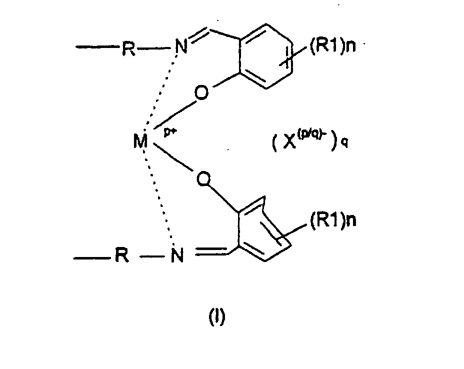 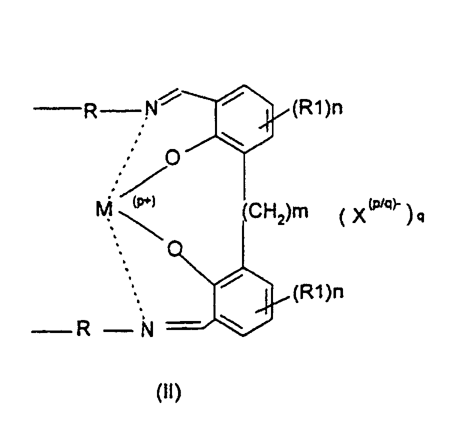 in which:
M is a metal ion or a combination of metal ions corresponding to the elements belonging to Groups IB to VIIB or VIII of Mendeleev's Periodic Classification (CAS version), including the lanthanides,
R is a linear or branched hydrocarbonaceous radical comprising from 1 to 12 carbon atoms,
R₁ is a halogen atom, a hydroxyl radical, an alkoxy radical, a carboxyl radical, an amine radical or an aliphatic, arylaliphatic, aromatic or alkylaliphatic hydrocarbonaceous radical which can comprise heteroatoms,
X is an anion,
n is an integer between 0 and 4,
m is an integer between 1 and 6,
p and q are integers between 0 and 4.

2. Process according to claim 1, **characterized in that** the metal element M is chosen from the group consisting of chromium, cobalt, iron, manganese, titanium, copper, vandium, molybdenum, ruthenium, gold and osmium.

3. Process according to claim 1, **characterized in that** the solid support is an inorganic oxide, chosen from the group consisting of alumina, silica, zirconium oxide, oxides of rare earth metals and titanium oxide, or inorganic compounds, such as lanthamum phosphate, or zeolites, molecular sieves or supports synthesized from functionalized polymers.

4. Process according to one of the preceding claims, **characterized in that** the catalyst is obtained by manufacture of a metal complex of general formulae III or IIIa: 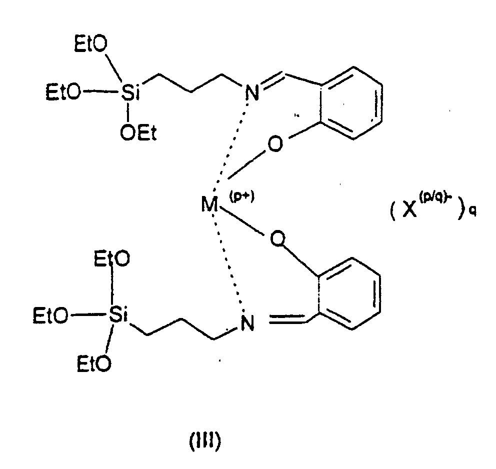 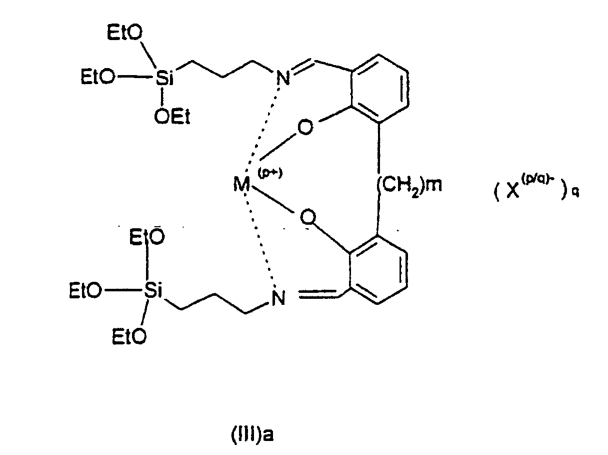 in a solvent and grafting the complex to the support by bringing the complex and the support into contact.

5. Process according to claim 4, **characterized in that** the solid support is produced in situ in the solution in the form of a gel.

6. Process according to one of the preceding claims, **characterized in that** the alkyl hydroperoxide is obtained by oxidation of an alkane by an oxidizing agent.

7. Process according to claim 6, **characterized in that** the oxidizing agent is chosen from oxygen, air or mixtures enriched in oxygen.

8. Process according to claim 6 or 7, **characterized in that** the alkane comprises from 3 to 30 carbon atoms and is chosen from the group consisting of propane, cyclohexane, cycloheptane, methylbenzene, ethylbenzene, phenylcyclohexane, diphenylmethane, phenylcyclododecane, cyclododecane, 1,2-dicyclohexylmethane, cumene, isobutane, 2-methylpropane, 2-propylbenzene, cyclohexene, 4-tert-butyl-1-cycloheptylbenzene, 2-isopropylnaphthalene, fluorene and 1,8-dimethylfluorene.

9. Process according to one of the preceding claims, **characterized in that** it comprises a stage of production of cyclohexyl hydroperoxide by oxidation of cyclohexane and decomposition of the hydroperoxide to cyclohexanone and/or cyclohexanol.

## Patentansprüche

1. Verfahren zur Herstellung einer Mischung von Ketonen/Alkoholen durch Zersetzung eines Alkylhydroperoxides in Anwesenheit eines Katalysators, umfassend ein katalytisch aktives metallisches Element, das auf einem festen Träger immobilisiert ist, **dadurch gekennzeichnet, daß** der Katalysator ein organometallisches Fragment der allgemeinen Formeln I oder II 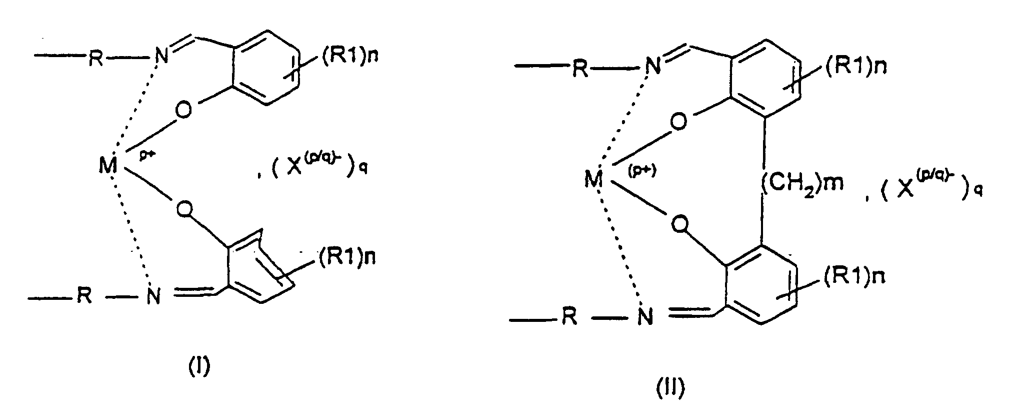 umfaßt, in denen
M ein metallisches Ion oder eine Kombination von metallischen Ionen darstellt, die den Elementen entsprechen, die aus den Gruppen IB bis VIIB oder VIII des Periodensystems von MENDELEEV (Version CAS) einschließlich der Lanthaniden stammen,
R ein linearer oder verzweigter Kohlenwasserstoff-Rest mit 1 bis 12 Kohlenstoffatomen ist,
R₁ ein Halogenatom oder einen Rest Hydroxyl, Alkoxy, Carboxyl, Amin oder einen aliphatischen, arylaliphatischen, aromatischen oder alkylaliphatischen Kohlenwasserstoff-Rest bedeutet, der Heteroatome umfassen kann,
X ist ein anion
n eine ganze Zahl zwischen 0 und 4 ist,
m eine ganze Zahl zwischen 1 und 6 ist,
p und q ganze Zahlen zwischen 0 und 4 sind.

2. Verfahren nach Anspruch 1, **dadurch gekennzeichnet, daß** das metallische Element M aus der Gruppe gewählt wird, die Chrom, Cobalt, Eisen, Mangan, Titan, Kupfer, Vanadium, Molybdän, Ruthenium, Gold und Osmium umfaßt.

3. Verfahren nach Anspruch 1, **dadurch gekennzeichnet, daß** der feste Träger ein mineralisches Oxid ist, gewählt aus der Gruppe, die Aluminiumoxid, Siliciumdioxid, Zirkoniumoxid, die Oxide der Seltenen Erden, Titanoxid oder mineralische Verbindungen wie Lanthanphosphat oder Zeolithe, Molekularsiebe oder ausgehend von funktionalisierten Polymeren synthetisierte Träger umfaßt.

4. Verfahren nach einem der vorstehenden Ansprüche, **dadurch gekennzeichnet, daß** der Katalysator durch Herstellung eines metallischen Komplexes der allgemeinen Formeln III oder IIIa 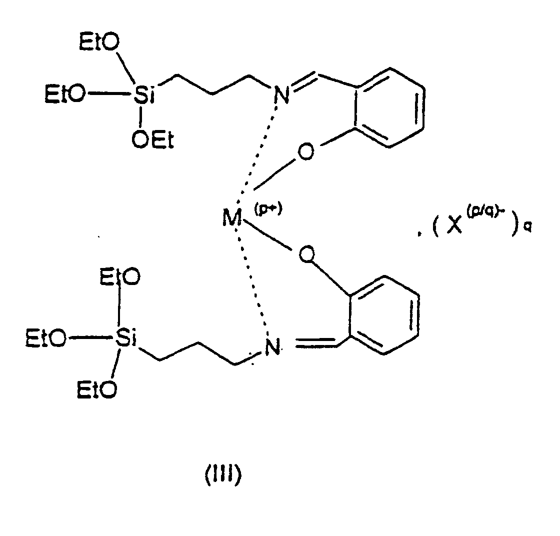 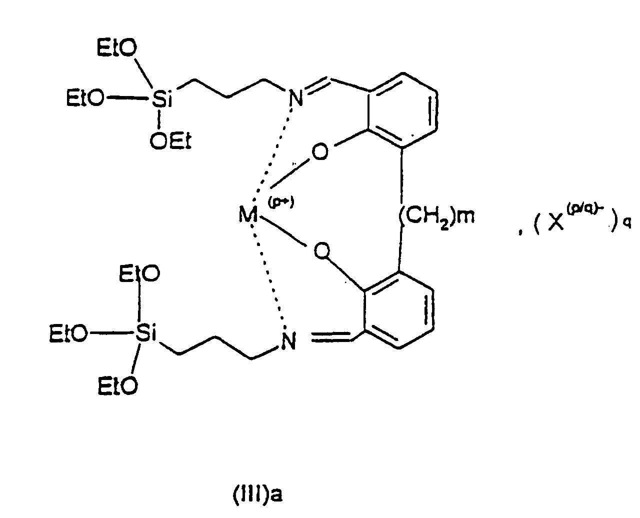 in einem Lösungsmittel und Pfropfung des Komplexes auf den Träger durch In-Kontakt-Bringen von Komplex und Träger erhalten wird.

5. Verfahren nach Anspruch 4, **dadurch gekennzeichnet, daß** der feste Träger in situ in der Lösung in Form von Gel hergestellt wird.

6. Verfahren nach einem der vorstehenden Ansprüche, **dadurch gekennzeichnet, daß** das Alkylhydroperoxid durch Oxidation eines Alkans durch ein Oxidationsmittel erhalten wird.

7. Verfahren nach Anspruch 6, **dadurch gekennzeichnet, daß** das Oxidationsmittel unter Sauerstoff, Luft oder den mit Sauerstoff angereicherten Mischungen ausgewählt wird.

8. Verfahren nach Anspruch 6 oder 7, **dadurch gekennzeichnet, daß** das Alkan 3 bis 30 Kohlenstoffatome enthält und aus der Gruppe gewählt wird, die Propan, Cyclohexan, Cycloheptan, Methylbenzol, Ethylbenzol, Phenylcyclohexan, Diphenylmethan, Phenylcyclododecan, Cyclododecan, 1,2-Dicyclohexylmethan, Cumol, Isobutan, 2-Methylpropan, 2-Propylbenzol, Cyclohexen, 4-tert.-Butyl-1-cycloheptylbenzol, 2-Isopropylnaphthalin, Fluoren oder 1,8-Dimethylfluoren umfaßt.

9. Verfahren nach einem der vorstehenden Ansprüche, **dadurch gekennzeichnet, daß** es eine Stufe der Herstellung von Cyclohexylhydroperoxid durch Oxidation von Cyclohexan und der Zersetzung des Hydroperoxids zu Cyclohexanon und/oder Cyclohexanol umfaßt.
